(19) Europäisches Patentamt
European Patent Office
Office européen des brevets

(11) Veröffentlichungsnummer: **0 599 152 A1**

# (12) EUROPÄISCHE PATENTANMELDUNG

(21) Anmeldenummer: **93118319.8**

(22) Anmeldetag: **12.11.93**

(51) Int. Cl.5: **C07D 487/14**, C07D 253/06, C07D 401/12, C07D 403/12, A61K 31/53, //(C07D487/14, 237:00,237:00)

(30) Priorität: **25.11.92 DE 4239540**

(43) Veröffentlichungstag der Anmeldung: **01.06.94 Patentblatt 94/22**

(84) Benannte Vertragsstaaten: **AT BE CH DE DK ES FR GB GR IE IT LI LU MC NL PT SE**

(71) Anmelder: **ASTA Medica Aktiengesellschaft**
**An der Pikardie 10**
**D-01277 Dresden(DE)**

(72) Erfinder: **Kutscher, Bernhard, Dr.**
**Stresemannstrasse 9**
**D-63477 Maintal(DE)**
Erfinder: **Engel, Jürgen, Prof.**
**Erlenweg 3**
**D-63755 Alzenau(DE)**
Erfinder: **Metzenauer, Peter Dr.**
**Taunusstrasse 9**
**D-63584 Gründau(DE)**
Erfinder: **Achterrath-Tuckermann, Ute, Dr.**
**Bahnhofstrasse 120 a**
**D-63477 Maintal 1(DE)**
Erfinder: **Szelenyi, Stefan, Prof.**
**Händelstrasse 32**
**d-90571 Schwaig(DE)**

## (54) Triazinone une Triazinophthalazinone mit antiasthmatischer/antiallergischer, positiv inotroper und blutdrucksenkender Wirkung.

(57) Verbindung des Triazinon-Typs mit der allgemeinen Formel 1, die antiasthmatische, antiallergische und kreislaufaktive Wirkungen haben sowie Verfahrensvarianten zu ihrer Herstellung wurden beschrieben.

EP 0 599 152 A1

Die Erfindung beschreibt neue Aryltriazinone und Aryltriazinophthalazine, Verfahren zur ihrer Herstellung und die Verwendung der erfindungsgemäßen Verbindungen zur Therapie von Asthma bronchiale, Allergien der unterschiedlichsten Genese, Entzündungsprozessen, Herzinsuffizienz und Hypertonie.

Die erfindungsgemäßen Verbindungen zeichnen sich beispielsweise als potente und selektive Hemmstoffe der 5-Lipoxigenase aus und können daher bei Krankheiten eingesetzt werden, bei denen eine Beteiligung der 5-Lipoxigenase oder der Leukotriene am pathophysiologischen Geschehen eine Rolle spielt. Sayed et al (Chinese Journal of Chemistry, No. 1, (1991) S. 45) beschrieben die Synthese von substituierten Phthalazinon-Derivaten durch die Umsetzung von Phthaliden oder substituierten Benzoylbenzoesäureestern mit Hydrazin. Die weitere Umsetzung führt zu 7-(p-Ethylphenyl)-2H-1,2,4-triazino [3,4-a] phthalazin-3-(4H)-onen als Nebenprodukt. Camparini et al (J. Heterocyclic Chem. 15, 1271 (1978) beschreiben die Umsetzung von Iminoestern und substituierten Hydrazinen zu 2,5-Dihydro-1,2,4-triazin 6(1H)-onen. Die Reaktion verläuft in guter Ausbeute.

Das EP-052 422 beschreibt Triazinone, die in der 6-Stellung durch einen substituierten aromatischen Ring substituiert sind. Die Verbindungen verfügen über cardiotonische Eigenschaften und eignen sich darüberhinaus zur Behandlung von Morbus Parkinson.

Die europäische Patentanmeldung 80 296 beschreibt durch aromatische Reste in 4-Stellung substituierte Oxadiazinone, Triazinone und Thiadiazinone mit cardiotonischen und antihypertensiven Eigenschaften.

Die erfindungsgemäßen Verbindungen lassen sich durch die Formel 1 beschreiben:

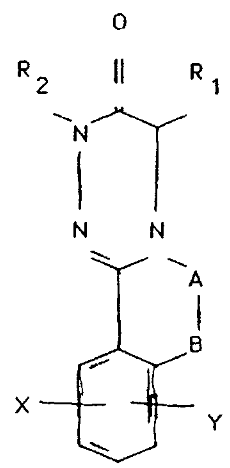

Die erfindungsgemäßen Verbindungen können auch in tautomeren Formen vorliegen.
Ein assymetrisches Kohlenstoffatom im Molekül läßt die erfindungsgemäßen Verbindungen in spiegelbildisomeren Formen auftreten. Die erfindudngsgemäßen Verbindungen können enantiomerenrein in der (R)- oder (S)-Form oder in Form des racemischen Gemisches oder in Gemischen beliebiger Zusammensetzung vorliegen.

Synthese der Verbindungen

Alle drei beschriebenen Verfahrensvariationen führen zu Verbindungen der allgemeinen Formel 1.

Verfahren 1

7-Phenyl-2H-triazino(3,4,a)phthalazin-3-(4H)one

Die erfindungsgemäßen Phenyltriazinophthalazinone gemäß Formel IV können entsprechend Schema I hergestellt werden.
Die Bedeutungen von X und Y entsprechen den obengenannten Definitionen.

Basierend auf prinzipiell literaturbekannten Methoden werden als Vorstufen 4-Arylphthalazinone gemäß Formel I (hergestellt entsprechend J. Pharmaceutical Soc. Jpn 86, 576 (1966) beziehungsweise USP 3,694,442) und durch Alkylierung (entsprechend J. Organic Chem. 50, 1677 (1985)) der Alkyl-4-Arylphthalazinon-acetate-gemäßFormel II hergestellt. Als Lösungsmittel im Schritt von Verbindung II nach III im Schema 1 kommen aromatische Kohlenwasserstoffe in Frage, die flüssig sind und die durch eine oder mehrere Alkylgruppen substituiert sein können wie beispielsweise Benzol, Toluol und die isomeren Xylole.

Nach Überführung in die Thioamide gemäß Formel III (mit $P_2S_5$ oder Lawesson-Reagenz) wird mit Hydrazin oder substituierten Hydrazin-Derivaten in Alkoholen bei 50°C - 80°C cyclisiert.

Unter dem Lawesson-Reagenz wird 2,4-(4-Methoxyphenyl)-1,3-dithia-2,4-diphosphetan-2,4-disulfid verstanden, das gemäß Synthesis Seite 941 (1979) oder gemäß T. P. Anderson et al, Tetrahedron, 39 (20), 2419 (1983) hergestellt werden kann.

Als Alkohole können aliphatische Alkohole, die ein oder mehrere Hydroxylgruppen tragen und eine Kettenlänge von 1-4 Kohlenstoffatomen aufweisen, verwendet werden. Es eignen sich beispielsweise Methanol, Ethanol, Propanol, Isopropanol und die isomeren Butanole.

Schema I

Synthese der Aryl-triazino-phthalazine

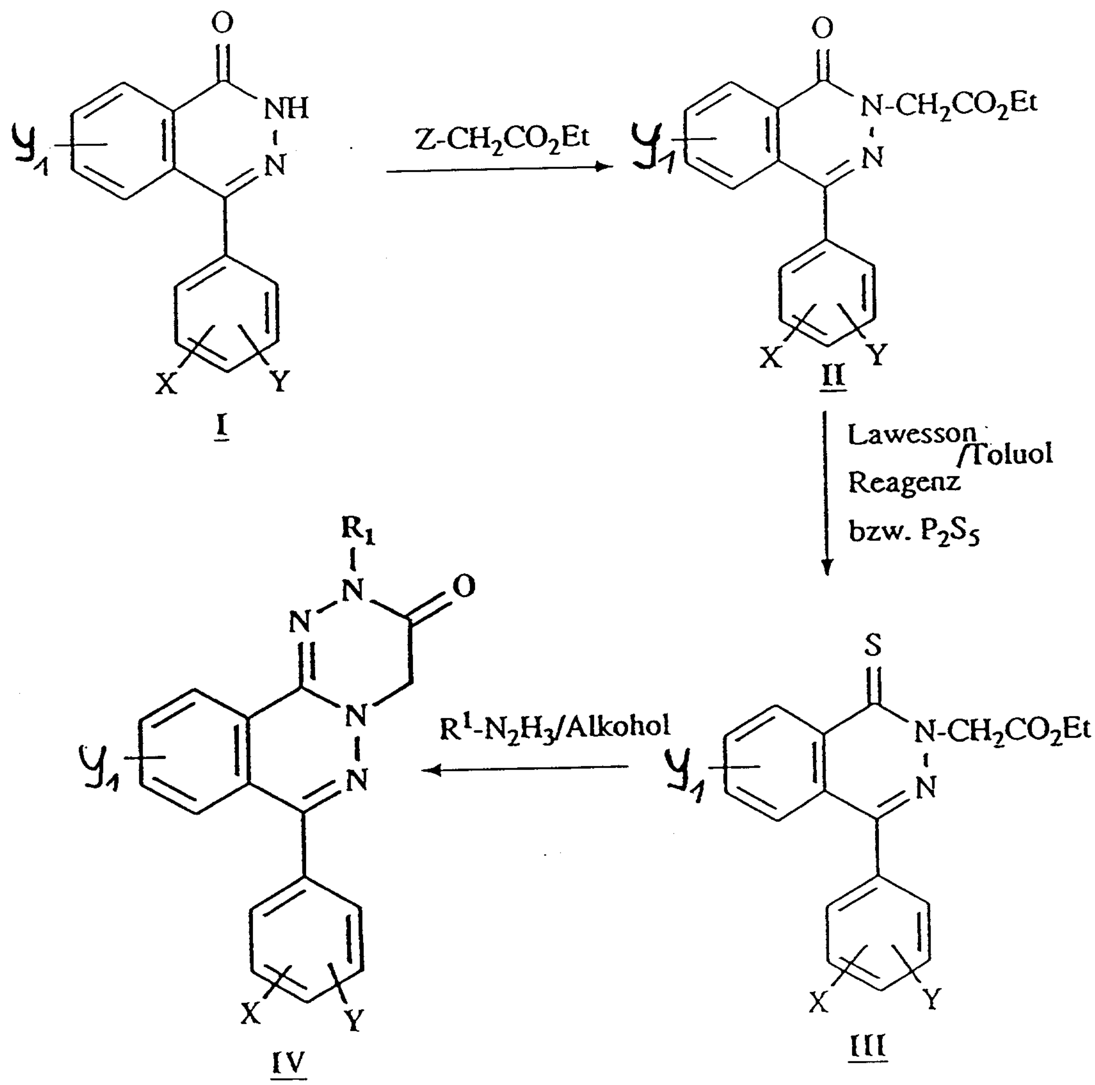


I, II, III, IV

Beispiel 1

7-Phenyl-2H-triazino(3,4a)phthalazin-3(4H)-on (D-19509)

7 g (0.032 mol) 4-Phenylphthalazinon (Smp.: 232-234°C) werden in 100 ml Toluol vorgelegt und portionsweise 0.8 g (0.036 mol) Natriumhydrid bei Raumtemperatur zugegeben. Anschließend wird 1 Stunde refluxiert und nach dem Abkühlen auf 50°C mit 5.4 g (0.032 mol) Bromessigsäureethylester versetzt. Es wird 5 h refluxiert, der ausgefallene Niederschlag abgetrennt und die Lösung eingeengt. Die ausgefallenen Kristalle werden in 200 ml Toluol aufgenommen und die Lösung mit 9 g (0.022 mal) Lawesson-Reagenz (2,4-Bis(4-methoxyphenl)-2,4-dithiooxo-1,3,2,4-dithiophosphetan) versetzt. Nach 24 h bei 80 C wird im Vakuum eingeengt und das ausfallende Thioamid isoliert, 8 g (0.025 mol) Thioamid werden in 100 ml Ethanol gelöst, 1.5 g (0.03 mol) Hydrazinhydrat zugegeben und 18 Stunden refluxiert. Der Niederschlag wird abgesaugt, in Wasser suspendiert und getrocknet. Schmelzpunkt: 251-252°C
DC (Chloroform/Methanol/25 % Ammoniaklösung 95/5/1) Rf = 0.69
Die nachfolgenden Verbindungen aus Tabelle 1 der Formel IV wurden entsprechend Beispiel 1 hergestellt.

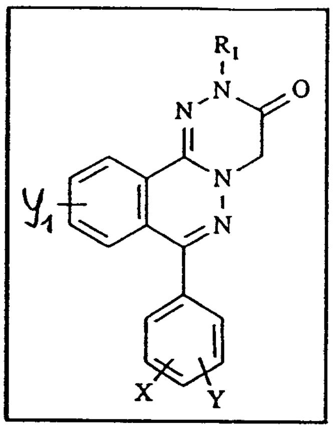

| B. Nr. | Verb. Formel IV X | Y | R1 | $y_1$ | Krist. aus | Smp. |
|---|---|---|---|---|---|---|
| 2 | 3-Methoxy | 4-Methoxy | H | H | Methanol | 249-252 °C |
| 3 | 3-Methoxy | 4-Methoxy | H | 7-Methyl | Methanol | 237-239 °C |
| 4 | 3-Oxyethylen | 4-Oxethylen | H | H | Eisessig | 266 °C |
| 5 | 3-Fluor | 4-Fluor | H | H | Ethanol | 269 °C |
| 6 | 4-Fluor | H | H | H | Butanol | 257-259 °C |
| 7 | 4-Methoxy | H | H | H | Dioxan | 266 °C |
| 8 | 4-OBzl | H | H | H | Dioxan | 272 °C |
| 9 | $N(CH_3)_2$ | H | H | H | Dioxan | 268 °C |
| 10 | 4-S-$CH_3$ | H | H | H | Eisessig | 282 °C |
| 11 | 3-Cl | 4-OH | H | H | Methanol | 275-277 °C |
| 12 | 4-OH | H | H | H | Butanol | 308 °C |
| 13 | 4-(2-Pyridylmethyl)oxy | Cl | H | H | Eisessig | 263-264 °C |
| 14 | 4-(2-Pyridylmethyl)oxy | 3-Cl | H | H | Eisessig | >300 °C |
| 15 | 4-(2-Chinolylmethyl)oxy | 3-Cl | H | H | Eisessig | >300 °C |

Verfahren 2

3-Phenyl-4,5-dihydro-1,2,4-triazin-6-one

Die erfindungsgemäßen Verbindungen können entsprechend Schema II hergestellt werden.
Substituierte 4-Hydroxy-beziehungsweise 4-Amino-benzoylaminosäureester gemäß Formel V (hergestellt entsprechend Literatur, zum Beispiel EP 422191 oder Bull. Chem. Soc. Chim. Belg., 92 (11), 1029 (1983))

dienen als Vorstufen.

Die Benzoylaminosäureester V werden in bekannter Weise mit Aryl- beziehungsweise Heteroarylalkylhalogeniden in organischen Lösungsmitteln wie Toluol oder DMA oder deren Gemischen unter Verwendung einer Hilfsbase in die entsprechenden Arylalkyl- beziehungsweise Heteroalkylether-, -thioether oder -amine des Typs VI überführt.

Als Hilfsbase können Alkalimetallcarbonate, Erdalkalimetallcarbonate und aliphatische Amine eingesetzt werden, beispielsweise Natriumcarbonat, Kaliumcarbonat oder Triethylamin.

Durch Aktivierung der Amidbildung im VI mit Hilfe der Bildung von Imidchloriden (unter Verwendung von $POCl_3/PCl_5$ oder $SOCl_2$ als Reagenz) oder Herstellung der Thioamide (durch Verwendung von $P_2S_5$ oder Lawesson-Reagenz) werden die Zwischenstufen VII erhalten.

Mit Hydrazin oder substituierten Hydrazinderivaten in $C_1$-$C_4$-Alkoholen, vorzugsweise Ethanol oder Butanol, werden die erfindungsgemäßen Verbindungen des Typs VII bei Temperaturen von 40°C-80°C erhalten.

Als substituierte Hydrazinderivate kommen Alkylhydrazine, wie zum Beispiel Methyl-, Ethylhydrazin und auch Benzylhydrazin in Betracht.

Beispiel 16

3-{3-Chlor-4-[(2-chinolyl)methoxy]phenyl}-4-methyl-4,5 -dihydro-1,2,4-triazin-6-on (D-20783)

a) N-(3-Chlor-4-hydroxy)-benzoyl-sarcosinethylester

Beispiel für eine Verbindung gemäß Formel V

172.6 g (1 Mol) 3-Chlor-4-Hydroxybenzoesäure, 153.6 g (1 Mol) Sarcosin-ethylester Hydrochlorid und 3 Mol Triethylamin werden in 1.5 l Dichlormethan vorgelegt und auf -10 C abgekühlt. 60 g (0.4 mol) 1-Hydroxybenzotriazol-Hydrat und 211 g (1.1 mol) N-(3-Dimethylaminopropyl)-N'-ethylcarbodiimid Hydrochlorid werden zugegeben und 1 h bei - 10 C nachgerührt. Es wird bei Raumtemperatur solange nachgerührt, bis im Dünnschichtchromatogramm keine Ausgangsprodukte mehr erkennbar sind. Anschließend wird die organische Lösung auf 4 l Wasser gegossen und die abgetrennte wässrige Phase noch 2 mal mit je 300 ml Dichlormethan extrahiert. Die organischen Phasen werden vereinigt und über Natriumsulfat getrocknet und eingeengt.

Das erhaltene Produkt (gelbes Öl) wird an der Ölpumpe getrocknet und danach als Rohprodukt weiter umgesetzt.

b) N-[3-Chlor-4-(2-chinolyl)methoxy] benzoyl-sarcosinethylester (2)

Beispiel für eine Verbindung der Formel VI

271.7 g (1 Mol) (1) und 195.4 g (1.1 Mol) Chlormethylchinolin werden in 600 ml Toluol und 200 ml Dimethylacetamid vorgelegt. 1.2 Mol Kaliumcarbonat-Pulver werden zugesetzt und die Suspension unter Rückfluß 6 Stunden erhitzt. Nach beendeter Reaktion wird der Rückstand abgesaugt und mit Toluol nachgewaschen. Das Filtrat wird eingeengt und ein dunkles Öl erhalten.

c) N-[3-Chlor-4-(2-chinolyl)-methoxylphenyl-thioxosarcosinethylester (3)

Beispiel für eine Verbindung gemäß der allgemeinen Formel VII

412.8 g (1 Mol) (2) wird in 1 l Toluol abs. vorgelegt und mit 202.2 g (0.5 mol) Lawesson-Reagenz ( = 2,4-Bis-(4-methoxyphenyl)-2,4-dithiooxo-1,3,2,4-dithiadiphoshetan) versetzt. Es wird 4 Stunden bei 80°C erhitzt. Die Lösung wird am Rotationsverdampfer eingeengt. Es wird ein braunes zähes Öl erhalten, das als Rohprodukt weiter umgesetzt wird.

d) 3-[3-Chlor-4-((2-chinolyl)methoxy phenyl]-4-methyl-4,5-dihydro-1,2,4-triazin-6-on (D-20783)

428.9 g (1 Mol) (3) wird in 1 l Ethanol vorgelegt und mit 65 g (1.3 mol) Hydrazin-Hydrat versetzt. Anschließend wird 6 bis 10 Stunden unter Rückfluß erhitzt. Die Reaktionslösung wird am Rotationsverdampfer eingeengt. Es wird ein brauner Kristallbrei erhalten, der mit 1.5 l Aceton versetzt und 1 Stunde bei Raumtemperatur gerührt wird. Das abgesaugte, mit Aceton gewaschene Rohprodukt wird getrocknet.

Ausbeute: 39,89 g (12 %), Schmelzpunkt: 205-209°C

NMR-Daten (500 MHz, $D_6$-DMSO): (ppm) 10,45 (s, 1H, NH), 7,6-8,5 (m, GH, Chinolyl-Rest) 7,55 (s, 1H, 2H-Aromat), 7,4 (d, 1H, GH-Aromat) 7,35 (a, 1H, 5H-Aromat), 5,55 (S, 2H, $OCH_2$) 3,8 (s, 2H, N-$CH_2$), 2,75 (s, 3H, N-$CH_3$).

Die Verbindungen VII der nachfolgenden Tabelle 2 können entsprechend Beispiel 16 hergestellt werden.

Die erfindungsgemäßen Verbindungen des Typs VII der 3-Aryl-4,5-dihydro-1,2,4-triazin-6-one können auch nach alternativen Synthesewegen hergestellt werden. Diese sind den Schemata IIb und IIc zu entnehmen und als beispielhaft anzusehen.

Schema II a

## Synthese der 3-aryl-4,5-dihydro-1,2,4-triazin-6-one

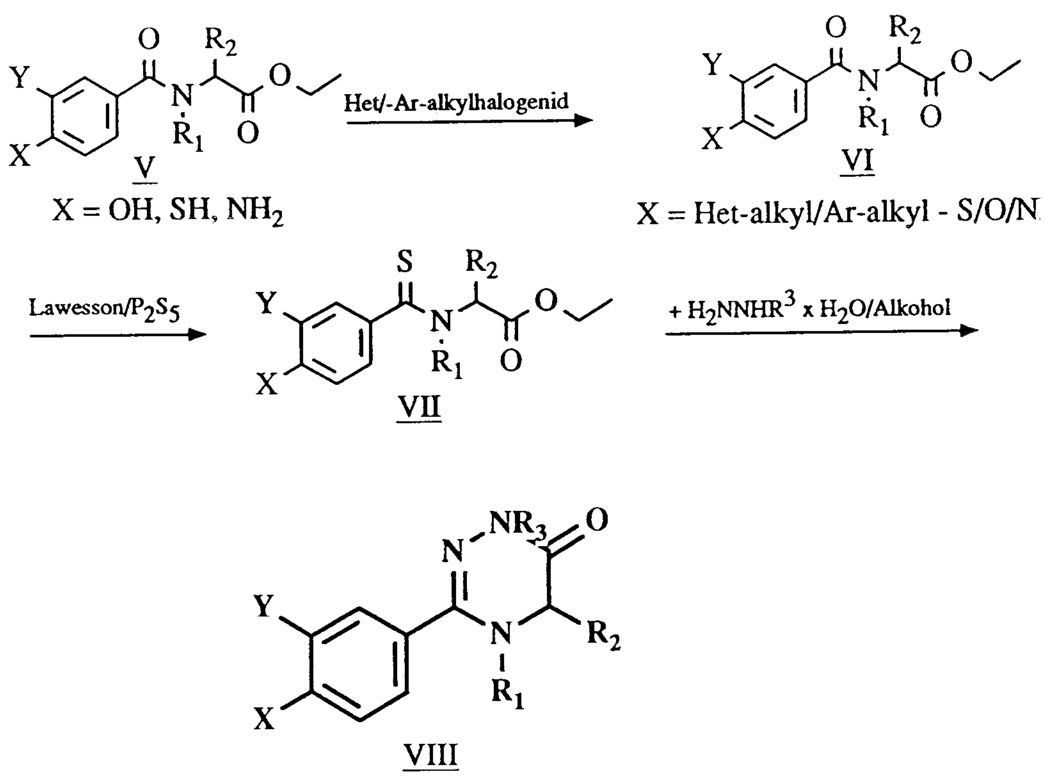


V

VI

VII

VIII

**Schema IIb**

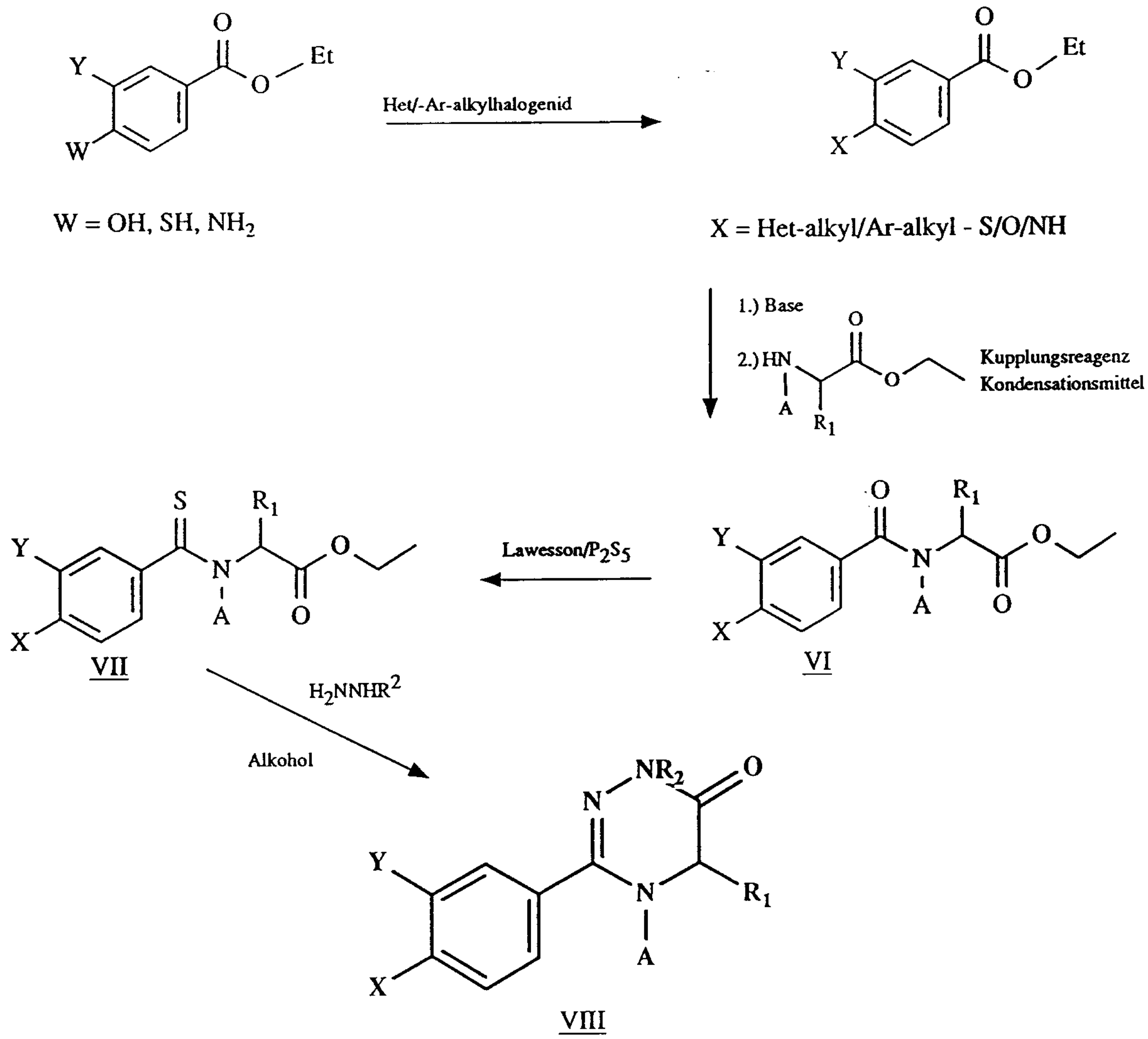


A = Alkyl $C_1$-$C_4$, Benzyl
$R_1$= H, Alkyl, Benzyl
$R_2$ = H, Alkyl, Benzyl

Schema IIc

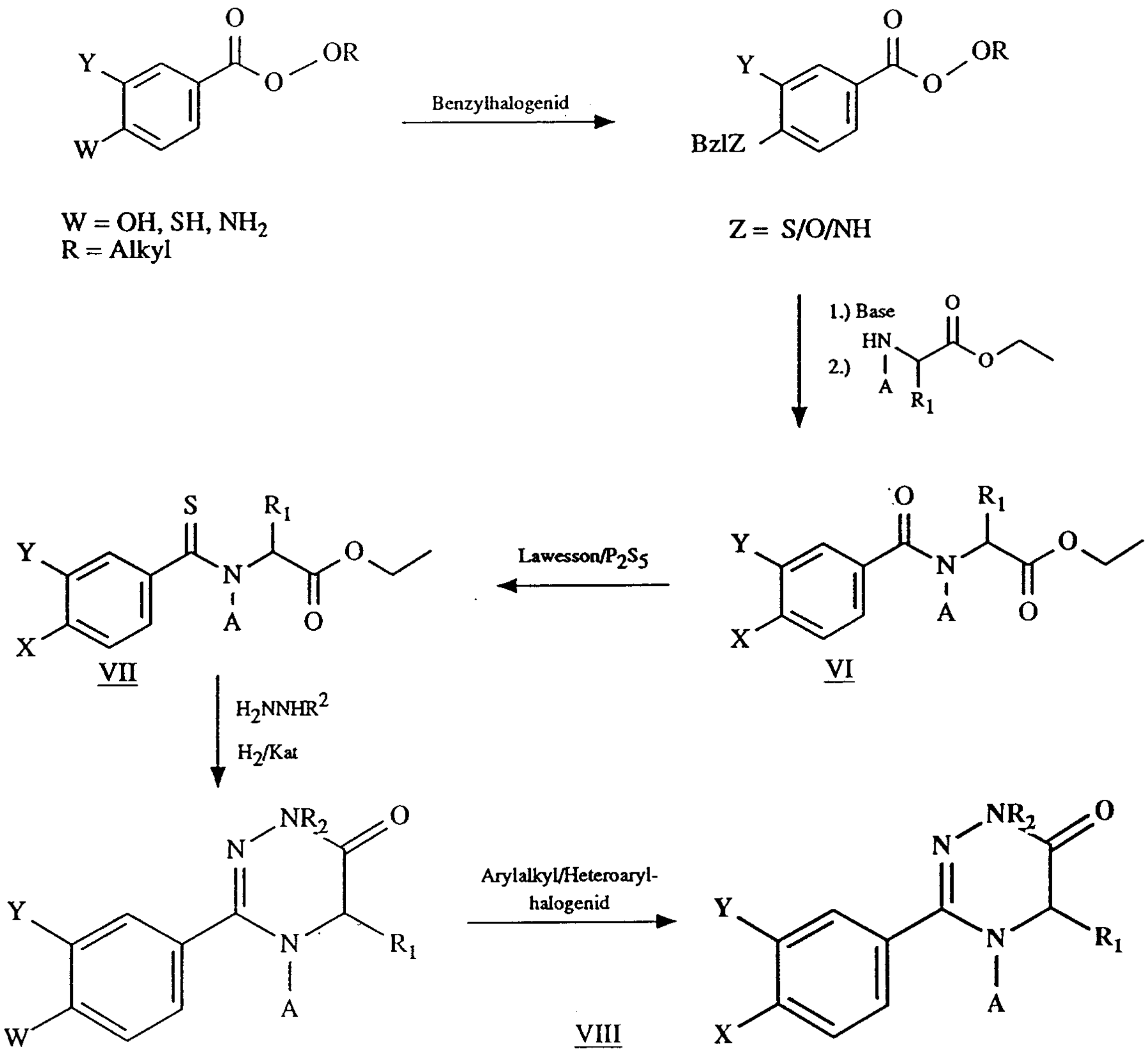


W = OH, SH, $NH_2$
R = Alkyl

Z = S/O/NH

VII

VI

VIII

A = Alkyl $C_1$-$C_4$, Benzyl
$R_1$= H, Alkyl, Benzyl
$R_2$ = H, Alkyl, Benzyl

Tabelle 2, Verbindungen gemäß Formel VII

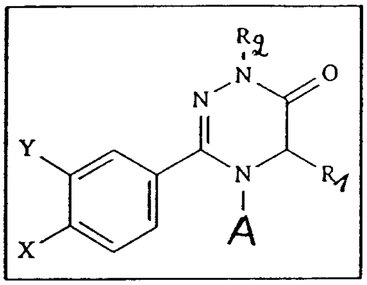

| B. Nr. | Verb. Formel VIII X | Y | A | $R_1$ | $R_2$ | Lösungsmittel | Smp. |
|---|---|---|---|---|---|---|---|
| 17 | OH | $NO_2$ | H | H | H | Eisessig | 242-244 °C |
| 18 | OH | $NH_2$ | H | H | H | Aceton | 213-215 °C |
| 19 | OH | H | H | H | H | Methanol | 249-251 °C |
| 20 | OH | Cl | $CH_3$ | H | H | Aceton | 228-231 °C |
| 21 | OH | Cl | H | H | H | Isopropanol/Methanol | 236-239 °C |
| 22 | OH | $OCH_3$ | H | H | H | Isopropanol/Methanol | 256-258 °C |
| 23 | OH | $OCH_3$ | H | H | H | Isopropanol/Methanol | 261-264 °C |
| 24 | OH | $OCH_3$ | H | $CH_3$ | H | Isopropanol | 244-247 °C |
| 25 | Bzl-O | H | H | H | H | Methanol | 199-202 °C |
| 26 | 3-[1-(2-Methoxyphenyl) -piperazinyl]-propyloxi | H | H | H | H | Isopropanol/Methanol | 133-135 °C |
| 27 | 3-[1-(2-Methoxyphenyl) -piperazinyl]-ethoxy | H | H | H | H | Isopropanol/Methanol | 139-141 °C |
| 28 | (2-Pyridylmethoxy) | H | H | H | H | Isopropanol | 178-182 °C |
| 29 | Bzl-O | H | $CH_3$ | H | H | Isopropanol/Methanol | 168-171 °C |
| 31 | 3-[1-(2-Methoxyphenyl) piperazinyl]-ethoxy | Cl | $CH_3$ | H | H | Iso propanol | 113-115 °C |

| B. Nr. | Verb. Formel VIII X | A | R1 | R2 | Lösungsmittel | Smp. |
|---|---|---|---|---|---|---|
| 32 | Bzl-O | $OCH_3$ | H | H | Isopropanol/Methanol | 231-233 °C |
| 33 | Bzl-O | Cl | $CH_3$ | H | Isopropanol | 252-254 °C |
| 34 | Bzl-O | Cl | H | H | Isopropanol | 241-243 °C |
| 35 | Bzl-O | $OCH_3$ | $CH_3$ | H | Isopropanol/Methanol | 212-214 °C |
| 36 | 3-Pyridylmethoxy | Cl | H | H | Isopropanol/Methanol | 246-249 °C |
| 37 | 3-Pyridylmethoxy | $OCH_3$ | H | H | Methanol | 243-245 °C |
| 38 | 3-Pyridylmethoxy | Cl, | $CH_3$ | H | Isopropanol | 158-162 °C |
| 39 | 3-Pyridylmethoxy | $OCH_3$ | H | H | Isopropanol | 186-188 °C |
| 40 | (4-Fluor)-Bzl-O | $OCH_3$ | H | H | Isopropanol | 189-191 °C |
| 41 | Bzl-O | $OCH_3$ | H | $CH_3$ | Isopropanol/Methanol | 228-231 °C |
| 42 | Bzl-S | F | H | H | Isopropanol/Methanol | 244-247 °C |
| 43 | Bzl-S | H | H | H | Isopropanol | 250-252 °C |
| 44 | Bzl-NH | $OCH_3$ | $CH_3$ | H | Isopropanol | 181-183 °C |
| 45 | Bzl-NH | H | H | H | Methanol | >250 °C |
| 46 | Bzl-S | F | $CH_3$ | H | Isopropanol | 138 °C |
| 47 | Bzl-S | $NH_2$ | $CH_3$ | H | Isopropanol/Methanol | 245-247 °C |
| 48 | Bzl-NH | $OCH_3$ | $CH_3$ | H | Isopropanol | 252-254 °C |
| 49 | 3-Pyridylmethylamino | H | $CH_3$ | H | Isopropanol | 238-241 °C |
| 50 | 3-Pyridylmethylamino | $OCH_3$ | $CH_3$ | H | Isopropanol/Methanol | 229-232 °C |

| B. Nr. | Verb. Formel VIII X | A | R1 | R2 | Lösungsmittel | Smp. |
|---|---|---|---|---|---|---|
| 51 | (4-Methoxy)benzyloxy | $OCH_3$ | H | H | Isopropanol | 198-201 °C |
| 52 | (4-Methoxy)benzyloxy | Cl | H | H | Isopropanol | 181-183 °C |
| 53 | 3-Pyridylmethylamino | $OCH_3$ | H | H | Methanol | 73-75 °C |
| 54 | 3-Pyridylmethylamino | H | H | H | Isopropanol | 231-233 °C |
| 55 | (2,3,4-Trimethoxy) -benzyloxy | Cl | H | H | Isopropanol/Methanol | 194-197 °C |
| 56 | 2-Chinolylmethoxy | Cl | $CH_3$ | H | Isopropanol/Methanol | 205-209 °C |
| 57 | 2-Chinolylmethoxy | $OCH_3$ | $CH_3$ | H | | 238-241 °C |
| 58 | 2-Chinolylmethoxy | Cl | H | H | Isopropanol/Methanol | 203-205 °C |

Verfahren 3

Beispiel 59

10-Methyl-3-Oxo-3,4-dihydro-2H-[1,2,4]triazino[4,3-c]chinazolin (D-19749)

Ausgehend von an sich bekannten Chinazolin-4-on-3-yl-essigsäureethylester-Derivaten gemäß Formel IX (s. M. Suisse, S. Johme, J. Prakt. Chem. (2), 326, 342 (1984) bzw. R. H. Clark, E.C. Wagner J. Org. Chem. 9, 55 (1944) werden die entsprechenden Chinolin-4-thion-3-yl-essigsäureethylester gemäß Formel X durch Beschwefelung mit $P_2S_5$ beziehungsweise Lawesson-Reagenz erhalten.

Zur Darstellung der Triazinochinazolinone gemäß Formel XI wurden die Thioamide gemäß Formel X mit Hydrazinhydrat in Alkoholen zu XI umgesetzt.

Als Alkohol kommen alle $C_1$-$C_4$-aliphatischen Alkohole in Frage, beispielsweise Methanol, Ethanol, Propanol, Isopropanol und die isomeren Butanole.

a) 6-Methylchinazolin-4-thion-3-yl-essigsäureethylester

Eine Suspension von 20 g (0,081 mol) 6-Methylchinazolin-4-on-3-yl-essigsäureethylester und 17,1 (0,042 mol) Lawesson-Reagenz in 300 ml Toluol wird 33 h bei 80 - 105°C gerührt, wobei sich langsam eine orang- bis dunkelrote Lösung bildet. Nach Reaktionsende wird die Lösung abgekühlt, eingeengt und der Rückstand mit wenig kaltem Toluol und anschließend mit Petrolether nachgewaschen und aus Dichlormethan/Methanol umkristallisiert. Fp: 130-132°C

b) 10-Methyl-3-oxo-3,4-dihydro-2H-[1,2,4]-triazino[4,3-c]chinazolin

8,7 g (0,033 mol) des Thioamids werden in 200 ml Ethanol suspendiert und mit 10,1 (0,2 mol) Hydrazinhydrat versetzt und die Mischung auf Rückflußtemperatur erwärmt. Nach vier Stunden wird der Niederschlag abgesaugt, mit Ethanol gewaschen und das Produkt aus Ethanol/Eisessig umkristallisiert. Fp: 298-304°C - Zersetzung

Entsprechend Beispiel 59 wurden die nachfolgenden Verbindungen der Tabelle erhalten.

Schema 3

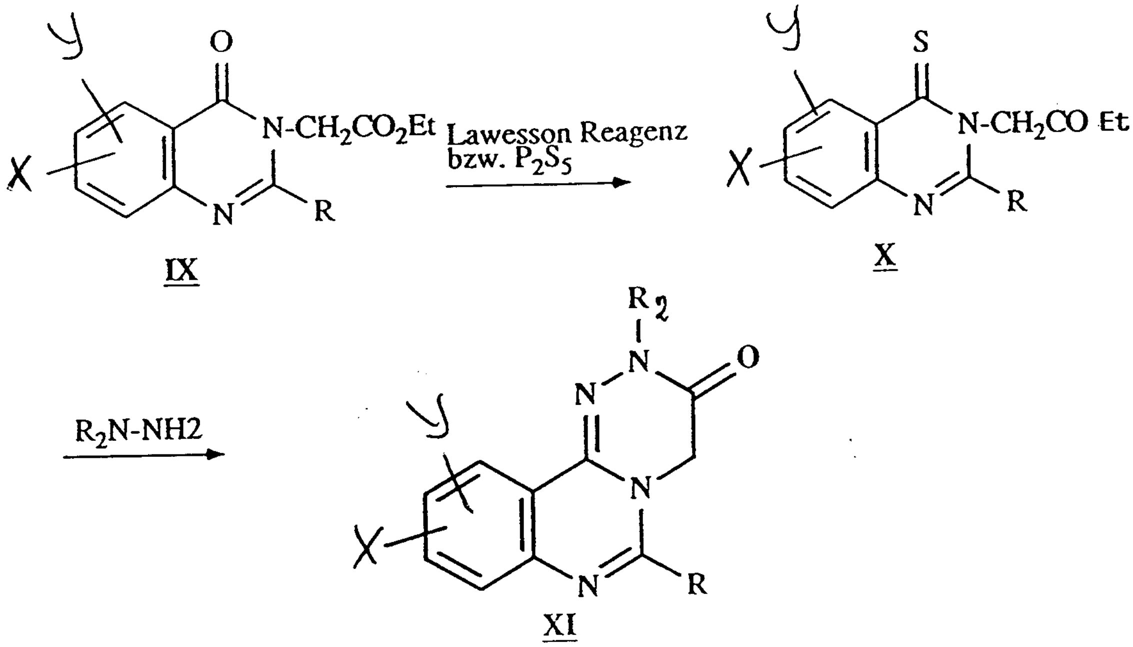


IX

X

XI

Tabelle 3, Formel XII

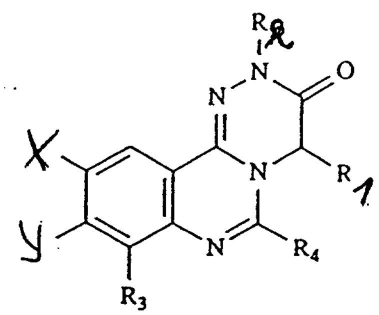

| B. Nr. | Verb. Formel XII X | Y | $R_1$ | $R_2$ | Lösungsmittel | Smp. |
|---|---|---|---|---|---|---|
| 60 | $CH_3$ | H | H | H | Ethanol/Eisessig | 298-304 °C |
| 61 | $CH_3$ | H | $CH_3$ | H | Ethanol/Eisessig | 265-269 °C |
| 62 | $OCH_3$ | $OCH_3$ | $OCH_3$ | H | Ethanol/Eisessig | 273-275 °C |
| 63 | Cl | H | H | H | Dichlormethan/Methanol | >300 °C |
| 64 | Cl | H | Cl | H | Ethanol/Eisessig | >300 °C |
| 65 | Cl | H | H | $CH_3$ | Ethanol/Eisessig | >300 °C |
| 66 | Cl | H | Cl | $CH_3$ | Ethanol/Eisessig | >300 °C |
| 67 | H | H | H | H | Ethanol | >300 °C |

Die erfindungsgemäßen Verbindungen wirken entzündungshemmend (Lipoxigenase-Hemmung) und bronchorelaxierend (Modell der mit Carbachol präkontrahierte Trachea des Meerschweinchens)

Weiter sind die Verbindungen kreislaufaktiv (Steigerung der linksventrikulären Kontraktilität, Blutdrucksenkung).

Die Verbindungen hemmen auch die durch Histamin induzierte Rhinitis an der Ratte sowie die Histamin-Freisetzung aus Mastzellen.

Die pharmakologische Testung auf Wirksamkeit bei der allergisch induzierten Histaminfreisetzung an sensibilisierten Ratten erfolgte gemäß der Versuchsvorschrift von P.A. Shore et al., I. Pharmacol. Exp. Ther., 127 (1959), S. 182.

Die muskelrelaxierende Wirkung der Substanzen wurde an mit Carbachol (INN) präkontrahierten Trachealketten von Meerschweinchen gemäß Tallandria et. al, Manual of Pharmacological Calculations with Computer programs, Springer, 1981 gefunden.

Die Hemmung der Produktion der Lipoxigenase-Folgeprodukte wurde an Ratten-Makrophagen gemäß der Vorschrift von Murphy et al, Methods in Enzymology 86, S. 409 - 416 vorgenommen.

Die Verbindung gemäß gemaß Beispiel 1 zeigt folgende Werte:

Bei der allergisch induzierten Histamin-Freisetzung ergibt ein Konzentration 10 µmol/l eine 25 % - Hemmung der Freisetzung.

Die Verbindung gemäß Beispiel 16 zeigt im Versuch der Hemmung der Spätphaseneosinophile in Meerschweinchen gemäß der Vorschrift von Cartwright, Diagnostic Laboratory Hematology, 1968, S. 48 und Mac Fortare et al, Eosinophil Counting, Brit. Med. 9., 2, 1187 (1951) in der Dosis von 30 mg pro Kilogramm

Körpergewicht, gegeben p.o., einen Hemmwert von 80 %.
Bei der Hemmung der LTD-4-Rezeptor-Bindung gemäß Borbe und Zierenberg, Pharmacopsychiatry 18, 314 319 (1985) ergab sich eine Hemmung von 50 % der Aktivität bei einer Konzentration von 7,4 µmol/l.

## Patentansprüche

**1.** Verbindungen der Formel I

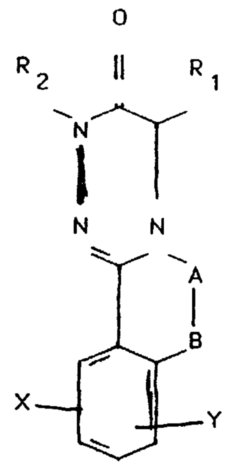

wobei gilt:
$R_1$ und $R_2$ können gleich oder verschieden sein, und Wasserstoff oder Alkylgruppen mit Kettenlängen von $C_1$-$C_4$, die auch verzweigt sein können, Benzylgruppen, die durch Halogenide, wie zum Beispiel Fluor, Chlor oder Brom substituiert sind, darstellen, weitere Substituenten der Benzylgruppe können $-CH_3$, $-NO_2$, Amino und substituierte Aminogruppen sein, des weiteren $-CF_3$; wenn $R_2$ = Benzyl oder substituiertes Benzyl ist, kann $R_2$ auch anneliert vorliegen,
A-B kann

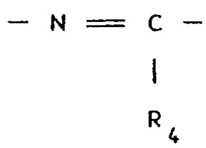 oder 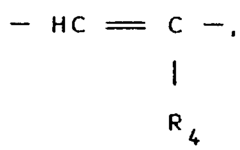 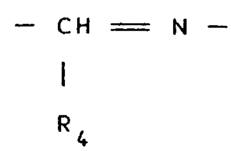

bedeuten, wobei gilt:

| | |
|---|---|
| $R_4$ | bedeutet Alkyl, verzweigtes Alkyl mit einer Kettenlänge von $C_1$ bis $C_4$, Phenyl, durch Halogenid und/oder Nitrogruppen einmal oder mehrfach substituiertes Phenyl, durch Alkylgruppen mit 3-6 Kohlenstoffatomen substituiertes Phenyl, die geradkettig oder verzweigt sein können, durch Alkoxygruppen, cyclische Alkoxy, Thiaalkyl-Chinuclidyl, durch Arylgruppen oder Heteroarylmethoxygruppen substituiertes Phenyl |
| A | kann Alkyl geradkettig oder verzweigt, mit einer Kettenlänge von 1-6 Kohlenstoffatomen oder Benzyl oder durch Halogen substituiertes Benzyl bedeuten, |
| B | kann Wasserstoff bedeuten, wenn gilt, daß zwischen A und B keine Bindung besteht. |
| X | kann Aryl oder Heteroaryl, Aryl oder Heteroarylmethoxy, Heteroarylmethylamino oder Heteroarylmethyltio bedeuten |
| Y | kann Halogen, Hydroxy, 0-Alkyl, mit einer Kettenlänge von 1-6 Kohlenstoffatomen, $NO_2$, $NR_5R_6$, wobei $R_5$ und $R_6$ gleich oder verschieden sein können und Wasserstoff, Alkyl, verzweigtes Alkyl mit 1-6 Kohlenstoffatomen sein können. |
| X und Y | können auch mehrfach vorkommen. |

**2.** Verfahren zur Herstellung der Verbindungen der Formel 1,
dadurch gekennzeichnet, daß man Verbindungen der allgemeinen Formel I mit Verbindungen der Formel Z-$CH_2$-C00 Et zu Verbindungen der Formel II umsetzt, anschließend mit Lawesson-Reagenz

oder $P_2$ $S_5$ zu Verbindungen der Formel III weiter umsetzt und mit sunbstituierten Hydrazinen zu Verbindungen der Formel IV umsetzt.

**3.** Verfahren zur Herstellung der Verbindungen der Formel 1, dadurch gekennzeichnet, daß man Verbindungen der allgemeinen Formel VI mit Lawesson-Reagenz oder $P_2S_5$ zu Verbindungen der allgemeinen Formel VII umsetzt und sie anschließend mit substituierten Hydrazinen zu Verbindungen der Formel VIII umsetzt.

**4.** Verfahren zur Herstellung der Verbindungen der Formel 1, dadurch gekennzeichnet, daß man Verbindungen der allgemeinen Formel IX mit Lawesson Reagenz oder $P_2S_5$ zu Verbindungen der Formel X umsetzt und anschließend mit substituierten Hydrazinen zu Verbindungen der Formel XI umsetzt.

**5.** Verwendung der Verbindung der Formel 1 zur Herstellung von Arzneimitteln.

**6.** Arzneimittel, dadurch gekennzeichnet, daß es Verbindung der Formel 1 in der Menge von 1 mg bis 1000 mg und die üblichen Zusatz- und Hilfsstoffe enthält.

**7.** Verfahren zur Herstellung von Arzneimitteln, dadurch gekennzeichnet, daß man Verbindungen der allgemeinen Formel 1 in der Menge zwischen 1 und 1000 mg mit den üblichen Trägern, Verbindungen und Hilfsstoffen in eine Formulierung verarbeitet.

**8.** Stoff, ausgewählt aus der Gruppe 7-Phenyl-2H-triazino[3,4-a]phthalazin-3(4H)on, 7-(3,4-Difluorphenyl)-2H-triazino[3,4-a]phthalazin -3(4H)on,
7-[4-(2-Pyridylmethoxy)-phenyl)-2H-triazino[3,4-a] -phthalazin-3(4H)on,
3-[3-Methoxy-4-(3-pyridylmethylamino)-phenyl]-4-methyl-4,5-dihydro-1,2,4-triazin-6-on,
3-[3-Chlor-4-(3-pyridylmethyloxy)-phenyl]-4-methyl-4,5-dihydro-1,2,4-triazin-6-on,
3-[3-Chlor-4-(3-pyridylmethyloxy)-phenyl]-4,5-dihydro-1,2,4-triazin-6-on,
3-[3-Methoxy-4-(3-pyridylmethoxy)-phenyl]-4,5-dihydro-1,2,4-triazin-6-on,
3-[3-Methoxy-4-(3-benzyloxy)-phenyl]-4-methyl-4,5-dihydro-1,2,4-triazin-6-on,
3-[3-Methoxy-4-(3-benzylmercapto)phenyl]-4-methyl-4,5-dihydro-1,2,4-triazin-6-on,
10-Methyl-3-oxo-3,4-dihydro-2H-[1,2,4]triazino-[4,3-c]chinazolin,
3-[3-Chlor-4-(2-chinolylmethoxy)-phenyl]-4-methyl-4,5-dihydro-1,2,4-triazin-6-on,
3-[3-Methoxy-4-(2-chinolylmethoxy)-phenyl]-4-methyl-4,5-dihydro-1,2,4-triazin-6-on,

**9.** Verwendung der Verbindungen gemäß Anspruch 8 zur Herstellung von Arzneimitteln.

**10.** Arzneimittel, dadurch gekennzeichnet, daß es eine oder mehrere Verbindungen gemäß Anspruch 8 in der Menge zwischen 1 mg und 1000 mg und die üblichen Zusatz- und Hilfsstoffe enthält.

**11.** Verfahren zur Herstellung von Arzneimitteln, dadurch gekennzeichnet, daß man eine oder mehrere Verbindungen gemäß Anspruch 8 in der Menge zwischen 1 mg und 1000 mg mit den üblichen Träger-, Konservierungs- und Hilfsstoffen zu einem Arzneimittel verarbeitet.

Europäisches Patentamt

# EUROPÄISCHER RECHERCHENBERICHT

Nummer der Anmeldung
EP 93 11 8319

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y | EP-A-0 123 254 (FUJISAWA PHARMACEUTICAL CO., LTD.) 10. Oktober 1990<br>* Seite 2, Zeile 27 - Seite 2, Zeile 31; Ansprüche 1-18 *<br>--- | 1-11 | C07D487/14<br>C07D253/06<br>C07D401/12<br>C07D403/12<br>A61K31/53<br>//(C07D487/14, 237:00,237:00) |
| Y | EP-A-0 238 357 (SANKYO CO. LTD.) 23. September 1987<br>* Seite 1, Zeile 13 - Seite 1, Zeile 21; Ansprüche 1-19 *<br>--- | 1-11 | |
| Y | EP-A-0 080 296 (ICI PLC) 1. Juni 1983<br>* Seite 2, Zeile 23 - Seite 2, Zeile 28; Ansprüche 1-10 *<br>--- | 1-11 | |
| Y | EP-A-0 383 449 (ORION-YHTYMÄ OY) 22. August 1990<br>* Seite 7, Zeile 29 - Seite 7, Zeile 41; Ansprüche 1-23 *<br>--- | 1-11 | |
| Y | EP-A-0 122 627 (FUJISAWA PHARMACEUTICAL CO., LTD.) 24. Oktober 1984<br>* Seite 26, Zeile 23 - Seite 26, Zeile 31; Ansprüche 1-14 *<br>--- | 1-11 | RECHERCHIERTE SACHGEBIETE (Int.Cl.5)<br>C07D<br>A61K |
| A | CHEMICAL ABSTRACTS, vol. 115, no. 5, 5. August 1991, Columbus, Ohio, US; abstract no. 49575y, M.A. SAYED ET AL. 'Synthesis and reactions of 4-aryl-1(2H)-phthalazinones'<br>Seite 836 ;<br>* Zusammenfassung *<br>& Chin. J. Chem. 9 (1) 45-53 (1991)<br>---<br>-/-- | 1-4,8 | |

Der vorliegende Recherchenbericht wurde für alle Patentansprüche erstellt

| Recherchenort | Abschlußdatum der Recherche | Prüfer |
|---|---|---|
| MÜNCHEN | 10. März 1994 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 93 11 8319

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | CHEMICAL ABSTRACTS, vol. 115, no. 3, 22. Juli 1991, Columbus, Ohio, US; abstract no. 29231e, M.A.E. SHABAN ET AL. 'Reactions of 4-aryl-1-hydrazinophthalazines with carbonyl compounds' Seite 767 ; * Zusammenfassung * & Pharmazie 46 (2) 105-8 (1991) --- | 1-4,8 | |
| A | CHEMICAL ABSTRACTS, vol. 100, no. 21, 21. Mai 1984, Columbus, Ohio, US; abstract no. 174786j, L.LEGRAND, N. LOZACH 'Heterocyclic sulfur compounds. CI. Reaction of methyl or ethyl hydrazinecarboxylates with 1,2-dihydro-3,1-benzothiazine-4-thiones' Seite 629 ; * Zusammenfassung * & Bull. Soc. Chim. Fr. 1983 (7-8) 226-9 ----- | 1-4,8 | |

RECHERCHIERTE SACHGEBIETE (Int.Cl.5)

Der vorliegende Recherchenbericht wurde für alle Patentansprüche erstellt

| Recherchenort | Abschlußdatum der Recherche | Prüfer |
|---|---|---|
| MÜNCHEN | 10. März 1994 | Herz, C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)